(19) Europäisches Patentamt
European Patent Office
Office européen des brevets

(11) Publication number: **0 074 255 A1**

# (12) EUROPEAN PATENT APPLICATION

(21) Application number: 82304641.2

(22) Date of filing: 03.09.82

(51) Int. Cl.³: C 07 D 263/04
A 01 N 25/32

(30) Priority: 08.09.81 US 300157

(43) Date of publication of application:
16.03.83 Bulletin 83/11

(84) Designated Contracting States:
AT BE CH DE FR GB IT LI NL

(71) Applicant: STAUFFER CHEMICAL COMPANY
Westport Connecticut 06880(US)

(72) Inventor: Caughan, Edmund Jeremiah
798 Wildcat Canyon Road
Berkeley California 94708(US)

(74) Representative: Smith, Sydney et al,
Elkington and Fife High Holborn House 52/54 High Holborn
London WC1V 6SH(GB)

## (54) Herbicide antidote, production, use and compositions thereof.

(57) A compound characterised in that it corresponds to the following formula:

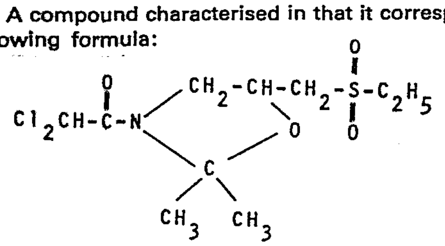

is disclosed, as is the production thereof.

This compound may be formulated with an inert diluent or carrier and/or adjuvant or with a thiocarbamate herbicide.

The use of the compound counteracts the crop damage commonly caused by herbicides and hence provides a method of establishing herbicidal selectivity.

EP 0 074 255 A1

"Herbicide antidote, production, use and compositions thereof"

This invention relates to a herbicide antidote, more particularly 2,2-dimethyl-3-(dichloroacetyl)-5-(ethylsulfonylmethyl)-1,3-oxazolidine, and to production, use and compositions thereof.

A herbicide is a compound which controls or modifies plant growth, e.g. killing, retarding, defoliating, desiccating, regulating, stunting, tillering, stimulating and dwarfing. The term "plant" refers to all physical parts of a plant, including seeds, seedlings, saplings, roots, tubers, stems, stalks, foliage and fruits. "Plant growth" includes all phases of development from seed germination to natural or induced cessation of life.

Herbicides are generally used to control or eradicate weed pests. They have gained a high degree of commercial success because it has been shown that such control may increase crop yield and reduce harvesting costs.

The most popular methods of herbicide application include: pre-plant incorporation into the soil; in-furrow application to seeds and surrounding soil; pre-emergence surface treatment of seeded soil; and post-emergence treatment of the plant and soil.

A manufacturer of herbicide generally recommends a range of application rates and concentrations calculated to maximize weed control. Generally, the range of rates varies from 0.01 to 50 pounds per acre (from 0.0111 to 56 kilograms per hectare (K/ha)), and is usually from 0.1 to 25 pounds per acre (from 0.112 to 28 k/ha). The term "herbicidally-effective amount" refers to the amount of a herbicide which controls or modifies plant growth. The actual amount used depends upon several considerations, including particular weed susceptibility and overall cost limitations.

The most important factor influencing the usefulness of a given herbicide is its selectivity towards crops. In some cases, a beneficial crop is susceptible to the effects of the herbicide. In addition, certain herbicidal compounds are phytotoxic to some weed species but not to others. To be effective, an herbicide must cause minimal damage (preferably no damage) to the beneficial crop while maximizing damage to weed species which plague that crop.

To preserve the beneficial aspects of herbicide use and to minimize crop damage, many herbicide antidotes have been prepared. These antidotes reduce or eliminate damage to the crop without substantially impairing the damaging effect of the herbicide on weed species; See, for example, U.S. Patent Nos. 3,959,304, 3,989,503, 4,021,224 and 4,021,229 and Belgian Patent No. 846,894.

The precise mechanism by which an antidote reduces herbicidal crop injury has not been established. An antidote compound may be a remedy, interferent, protectant, or antagonist. As used herein, "antidote" describes a compound which has the effect of establishing herbicide selectivity, i.e., continued herbicidal phytotoxicity to weed species and reduced or non-phytotoxicity to cultivated crop species. The term "antidotally effective amount" describes the amount of an antidote compound which counteracts a phytotoxic response of a beneficial crop to an herbicide.

Thiocarbamate herbicides are particularly effective in the control of grassy type weeds which interfere with the cultivation of a wide variety of crops, e.g., barley, corn, cotton, lentils, peanuts, peas, potatoes, soybeans, spinach, tobacco and tomatoes. Frequently the effective use of these herbicides requires the addition of an antidote compound.

## Description of the Invention

It has now been discovered that 2,2-dimethyl-3-(dichloroacetyl)-5-(ethylsulfonylmethyl)-1,3-oxazolidine is an effective antidote for the protection of a variety of crops from thiocarbamate herbicide injury. This compound has the following formula:

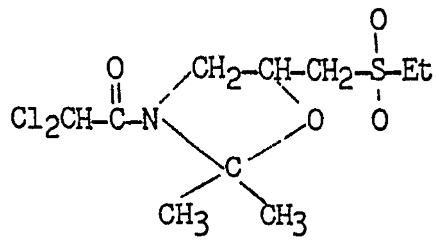

This invention also embodies a two-part herbicidal system comprised of:

(a) an herbicidally effective amount of a thiocarbamate compound of the formula:

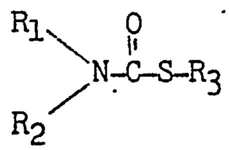

in which

$R_1$ is alkyl having 1-6 carbon atoms, inclusive;

$R_2$ is selected from the group consisting of alkyl having 1-6 carbon atoms, inclusive; and cyclohexyl; or

$R_1$ and $R_2$ form indistinguishable parts of a single alkylene ring having 4-10 carbon atoms, inclusive; and

$R_3$ is selected from the group consisting of alkyl having 1-6 carbon atoms, inclusive; haloalkyl wherein halo is selected from the group consisting of chlorine, bromine and iodine and alkyl has 1-6 carbon atoms, inclusive; alkenyl having 2-6 carbon atoms, inclusive; halo alkenyl wherein halo is selected from the group consisting of chlorine, bromine and iodine and alkenyl has 2-6 carbon atoms, inclusive; benzyl; and halo-substituted benzyl, wherein halo is selected from the group consisting of chlorine, bromine and iodine; and

(b) an non-phytotoxic antidotally effective amount of a compound of the formula

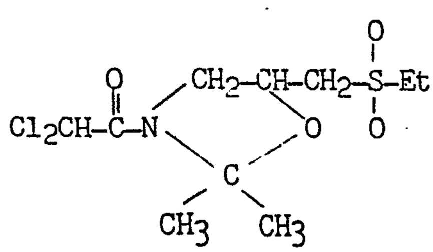

By way of exemplification, the active thiocarbamate herbicides employed in the invention may include: S-ethyl N,N-dipropyl thiocarbamate, S-ethyl N,N-diisobutyl thiocarbamate, S-propyl N,N-dipropyl thiocarbamate, S-propyl N,N-butylethylthiocarbamate, S-(2,3,3-tri- chloroallyl) N,N-diisopropyl thiocarbamate, S-ethyl N-ethyl N-cyclohexyl thiocarbamate, S-ethyl hexahydro- 1H-azepine-1-carbothioate, isopropyl hexahydro-1,4-azepine-1-carbothioate, S-benzyl N,N-disec-butylthiolcarbamate, S-(4-chlorobenzyl) N,N-diethyl thiolcarbamate and combinations thereof.

This invention also includes the method of establishing herbicidal selectivity which comprises applying to the locus where protection is desired an antidotally-effective amount of a compound of the formula

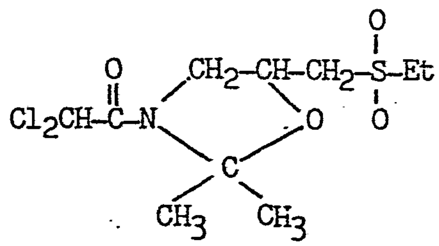

The locus where selectivity is desired may include soil, seeds, seedlings, and vegetation.

Preparation

The thiocarbamates of the present composition are either commercially available or can be prepared by the procedures described in U.S. Patent Nos. 2,913,327; 2,983,747; 3,133,947; 3,185,720; and 3,198,786..

The 2,2-dimethyl-3-(dichloroacetyl)-5-(ethylsulfonylmethyl)-1,3-oxazolidine compound of the present invention can be prepared according to the follow procedure.

Preparation of 2,2-dimethyl-3-(dichloroacetyl)-5-(ethylsulfonylmethyl)-1,3-oxazolidine

A solution containing 8.3 grams (g) (0.041 mole, 85% acid) of m-chloroperbenzoic acid in 100 milliliters (ml) of dichloromethane was prepared and warmed to 30°C. Five and six-tenths g of 2,2-dimethyl-3-(dichloroacetyl)-5-(ethylthiomethyl)-oxazolidine in 10 ml of

dichloromethane was added to the solution and the temperature rose to reflux. The mixture was refluxed for an hour, cooled to -5°C and filtered. The filtrate was washed three times with a sodium bicarbonate solution, once with water and once with a saturated sodium chloride solution. The filtrate was dried and the solvent was removed to leave a white solid which was titurated with hexane and dried. Yield was 4.1 g of 2,2-dimethyl-3-(dichloroacetyl)-5-(ethylsulfonylmethyl)-1,3-oxazolidine. m.p. = 121-126°C. Structure was confirmed by infrared spectrum.

Testing

Stock solutions of the herbicides were prepared by diluting the requisite amount of each herbicide in water. Examples of solution compositions and application rates are summarized in Table I.

TABLE I

Herbicide Stock Solutions

| Herbicide Name | Composition: Herbicide (mg)* | Composition: Water (ml) | Application: ml/flat** | Application: lb/acre |
|---|---|---|---|---|
| VERNAM® 6E | 390 | 400 | 5 | 1.00 |
| S-propyl-N,N-dipropyl thiocarbamate | 2340 | 400 | 5 | 6.00 |
| EPTAM® 6E S-ethyl-N,N-dipropyl thiocarbamate | 954 | 150 | 5 | 6.00 |

* The weight is measured in terms of mg of formulated herbicide. The formulations used contained about 72% active herbicide compound.

** The flats measure 5.95 inches by 9.5 inches. Approximately four (4) mg/flat is equal to one (1) lb/acre.

The herbicide was either incorporated into the soil prior to planting or applied to the soil after planting and prior to emergence of the plants. In some cases of pre-plant incorporation, the herbicide was incorporated into the soil alone in preparation for in-furrow application of the antidote; in others the herbicide solution was tank-mixed with the antidote solution prior to incorporation.

Stock solutions of the antidote compound were prepared at the desired concentrations by diluting the requisite amounts of each antidote in acetone. Examples of solution compositions, rates and application methods are summarized in Table II.

TABLE II

Antidote Stock Solutions

Antidote: 2,2-Dimethyl-3-(dichloroacetyl)-5-(ethylsulfonylmethyl) 1,3-oxazolidine

| Composition | | Application | | |
|---|---|---|---|---|
| Antidote (mg) | Acetone (ml) | ml/flat | lb/acre | Method |
| 95 | 15 | 0.30 | 1.00 | IF* |
| 20 | 20 | 2.00 | 0.50 | PPI** |

*IF = In-furrow surface application of antidote.

** PPI = Pre-plant incorporation

The antidote solutions were applied to the soil either by in-furrow surface application, or by pre-plant incorporation. In all cases of pre-plant incorporation, the antidote was tank-mixed with the herbicide prior to incorporation into the soil.

For in-furrow application, a one pint (473 cubic centimeter (cc)) sample of soil containing the previously incorporated herbicide was removed and retained from each planting flat. After leveling and furrowing the soil, seeds of the crop or weed species were planted 1/2 inch deep (1.27 centimeter). Each flat was divided in half by a wooden barrier. A stock solution of the antidote was atomized directly onto the exposed seeds and soil in the open furrow on one side of the bar rier. The seeds in the entire flat were then covered with the previously removed soil. The antidotally untreated sections of flats were compared for observed differences which would indicate lateral movement of the antidote through the soil.

Control flats contained crops treated with herbicide only.

All flats were placed on greenhouse benches where temperature was maintained between 70 and 90°F (21.1 to 32.2°C). The flats were watered by sprinkling as needed to assure good plant growth.

All of the soil used in the tests described herein was loamy sand soil treated with 50 parts per million (ppm) each of a commercially available fungicide, N-[(trichloromethyl)-thio]-4-cyclohexene-1,2-dicarboximide, and 18-18-18 fertilizer, which contains 18% by weight equivalent each of nitrogen, phosphorus pentoxide, and potassium oxide.

Injury ratings were taken four weeks after application of the antidote. The effectiveness of the antidote was determined by visual comparison of crop injury which occurred in the test flats to that which occurred in the control flats.

The treated crops initially screened for herbicidal injury were milo, wheat, cotton, rice, barley, corn and soybeans. The compound was tested on the weed species watergrass (Echinochloa crusgalli), foxtail (Setaria viridis) and shattercane (Sorghum bicolor).

KEY TO TABLES III AND IV

Herbicides

VERNAM® - S-propyl-N,N-di-propyl thiocarbamate
EPTAM® - S-ethyl-N,N-dipropyl thiocarbamate
RONEET® - S-ethyl N-ethyl-N-cyclohexyl thiocarbamate

Application Methods

PES = Surface application of herbicide to soil after planting of seeds and prior to emergence of plants.

IF = In-furrow surface application of antidote (soil previously treated with herbicide only).

PPI = Pre-plant incorporation of herbicide or antidote. If both herbicide and antidote were preplant incorporated, a tank-mixed solution was used.

TM = Tank-mixed solution of herbicide and antidote.

If no antidote was applied, the word "none" appears in the Antidote Rate column. These are the control flats for each crop. The results shown on this line are the percent injuries sustained by each of the crops when treated with the herbicide only at the rate specified.

All rates shown, for both herbicide and antidote, are in pounds per acre.

## Injury Ratings

The injury to the crop (Table III) or weeds (Table IV) is shown as a percentage of damage done to the plants as compared to an evaluation of the overall undamaged state of the plants. The damage done to the plants is a function of the number of plants injured and the extent of injury to each plant. This rating is made four (4) weeks after application of the herbicide alone or of the herbicide in combination with the antidote.

An asterisk (*) in Table III indicates that the antidote compound is active in reducing herbicidal injury to the crop. Parentheses around a number indicate that the test has been run more than once and the results are inconclusive.

Table IV shows that the antidote compound has no effect on weeds, i.e., herbicidal injury to the weeds is sustained even in the presence of the antidote compound.

TABLE III
Antidotal Effectiveness

| Herbicide | Herbicide Rate | Herbicide Method | Antidote Rate | Antidote Method | Milo % Inj | Wheat % Inj | Cotton % Inj | Rice % Inj | Barley % Inj | Corn % Inj | Soybean % Inj |
|---|---|---|---|---|---|---|---|---|---|---|---|
| VERNAM | 1.00 | PPI | none | - | 90 | 95 | 55 | 98 | 85 | | |
| VERNAM | 1.00 | PPI | 5.00 | IF | *15 | 95 | *50 | *90 | 85 | | |
| VERNAM | 6.00 | PPI | none | - | | | | | | 90 | 55 |
| VERNAM | 6.00 | PPI | 5.00 | IF | | | | | | * 0 | (35) |
| VERNAM | 6.00 | PPI | none | - | | | | | | | 50 |
| VERNAM | 6.00 | PPI | 1.00 | IF | | | | | | | 50 |
| VERNAM | 6.00 | PPI | 5.00 | IF | | | | | | | (50) |
| EPTAM | 6.00 | PPI | none | - | | | | | | 85 | |
| EPTAM | 6.00 | PPI/TM | 0.0125 | PPI/TM | | | | | | 0 | |
| EPTAM | 6.00 | PPI/TM | 0.025 | PPI/TM | | | | | | * 0 | |
| EPTAM | 6.00 | PPI/TM | 0.05 | PPI/TM | | | | | | * 0 | |
| EPTAM | 6.00 | PPI | none | - | | | | | | 97 | |
| EPTAM | 6.00 | PPI/TM | 0.005 | PPI/TM | | | | | | 95 | |
| EPTAM | 6.00 | PPI/TM | 0.0067 | PPI/TM | | | | | | 95 | |
| EPTAM | 6.00 | PPI/TM | 0.01 | PPI/TM | | | | | | *90 | |
| EPTAM | 6.00 | PPI/TM | 0.02 | PPI/TM | | | | | | *75 | |
| RONEET | 3.00 | PPI | none | - | 80 | | | | | | |
| RONEET | 3.00 | PPI | 0.50 | PPI/TM | *65 | | | | | | |
| RONEET | 3.00 | PPI/TM | 1.00 | PPI/TM | *50 | | | | | | |
| RONEET | 3.00 | PPI/TM | 2.00 | PPI/TM | *50 | | | | | | |
| RONEET | 3.00 | PPI/TM | 5.00 | PPI/TM | *40 | | | | | | |
| RONEET | 3.00 | PPI | none | - | 90 | | | | | | |
| RONEET | 3.00 | PPI | 1.00 | IF | *40 | | | | | | |
| RONEET | 3.00 | PPI | 5.00 | IF | *50 | | | | | | |

TABLE IV

Herbicidal Effectiveness

| Herbicide Name | Herbicide Rate | Herbicide Method | Antidote Rate | Antidote Method | Water-Grass | Foxtail | Shatter-cane |
|---|---|---|---|---|---|---|---|
| VERNAM | 6.00 | PPI | none | - | 100 | 100 | |
| VERNAM | 6.00 | PPI | 1.00 | IF | 100 | 100 | |
| VERNAM | 6.00 | PPI | 5.00 | IF | 100 | 100 | |
| EPTAM | 6.00 | PPI | none | - | 97 | 97 | |
| EPTAM | 6.00 | PPI/TM | 0.005 | PPI/TM | 97 | 97 | |
| EPTAM | 6.00 | PPI/TM | 0.0062 | PPI/TM | 97 | 97 | |
| EPTAM | 6.00 | PPI/TM | 0.01 | PPI/TM | 97 | 97 | |
| EPTAM | 6.00 | PPI/TM | 0.02 | PPI/TM | 97 | 97 | |
| RONEET | 3.00 | PPI | none | - | | 90 | 99 |
| RONEET | 3.00 | PPI/TM | 0.50 | PPI/TM | | 90 | 99 |
| RONEET | 3.00 | PPI/TM | 1.00 | PPI/TM | | 90 | 99 |
| RONEET | 3.00 | PPI/TM | 2.00 | PPI/TM | | 90 | 99 |
| RONEET | 3.00 | PPI/TM | 5.00 | PPI/TM | | 90 | 99 |
| RONEET | 3.00 | PPI | none | - | | 100 | 100 |
| RONEET | 3.00 | PPI | 1.00 | IF | | 100 | 100 |
| RONEET | 3.00 | PPI | 5.00 | IF | | 100 | 100 |

Test Results

2,2-Dimethy 1-3-(dichloroacetyl)-5-(ethylsulfonylmethyl)-1,3-oxazolidine shows good antidotal activity for a variety of crops. Use of this compound did not result in a reduction of herbicidal injury to weeds.

Formulations

A formulation is the incorporation of a formulant in a form which is directly usable on crops and weeds. As defined herein, a "formulant" is the material which is to be formulated. The formulant may be either an antidote compound alone or an herbicide and antidote composition. The purpose of the formulation is to apply the formulant to the locus where it is desired to establish herbicidal selectivity by a convenient method. The "locus" may include soil, seeds, seedlings and vegetation.

The formulations are commonly dusts, wettable powders, granules solutions or emulsifiable concentrates.

Dusts are free-flowing powder compositions containing the formulant impregnated on a particulate carrier. The particle size of the carriers is usually in the approximate range of 30 to 50 microns. Examples of suitable carriers are talc, bentonite, diatomaceous earth, and pyrophyllite. The composition generally contains up to 50% of formulant. Anti-caking and anti-static agents may also be added. Dusts may be applied by spraying from boom and hand sprayers on airplanes.

Wettable powders are finely divided compositions comprising a particulate carrier impregnated with the formulant and additionally containing one or more surface active agents. The surface active agent promotes rapid dispersion of the powder in an aqueous medium to form stable, sprayable suspensions. A wide variety of surface active agents can be used, for example, long chain fatty alcohols and alkali metal salts of the sulfated fatty alcohols; salts of sulfonic acid; esters of long chain fatty acids; and polyhydric alcohols, in which the alcohol groups are free, omega-substituted polyethylene glycols of relatively long chain length. A list of surface active agents suitable for use in agriculture formulations can be found in Wade Van Valkenburg, Pesticide Formulations (Marcel Dekker, Inc., N.Y., 1973) at pages 79-84.

Granules comprise the formulant impregnated on a particulate inert carrier having a particle size of about 1 to 2 millimeters (mm) in diameter. The granules can be made by spraying a solution of the formulant in a volatile solvent onto the granular carrier. Examples of suitable carriers for the preparation of granules include clay, vermiculite, sawdust, and granular carbon.

Emulsifiable concentrates consist of an oil solution of the formulant plus an emulsifying agent. Prior to use the concentrate is diluted with water to form a suspended emulsion of oil droplets. The emulsifiers used are usually a mixture of anionic and nonionic surfactants. Other additives, such as suspending agents and thickeners, may be included in the emulsifiable concentrate.

When the formulant is an antidote and herbicide composition, the proportion of antidote compound to herbicide compound generally

ranges from approximately 0.001 to 30 parts by weight of the antidote compound per weight of the herbicide compound.

Formulations generally contain several additives in addition to the formulant and carrier or agent. Among these are inert ingredients, diluent carriers, organic solvents, water, oil and water, water in oil emulsions, carriers of dusts and granules, and surface active wetting, dispersing and emulsifying agents. Fertilizers, e.g., ammonium nitrate, urea and superphosphate, may be included. Aids to rooting and growth, e.g., compost, manure, humus and sand, may also be included.

Alternatively, the antidote compounds and herbicide and antidote compositions of this invention can be applied to a crop by addition of the formulant to irrigation water supplied to the field to be treated. This method of application permits the penetration of the compositions into the soil as the water is absorbed.

As another alternative, the formulant can be applied to the soil in the form of a solution in a suitable solvent. Solvents frequently used in these formulations include kerosene, fuel oil, xylene, petroleum fractions with boiling ranges above xylene and aromatic petroleum fractions rich in methylated naphthalenes. Liquid solutions, like dusts, may be applied by spraying from boom and hand sprayers on airplanes.

CLAIMS:

1. A compound characterized in that it corresponds to the following formula:

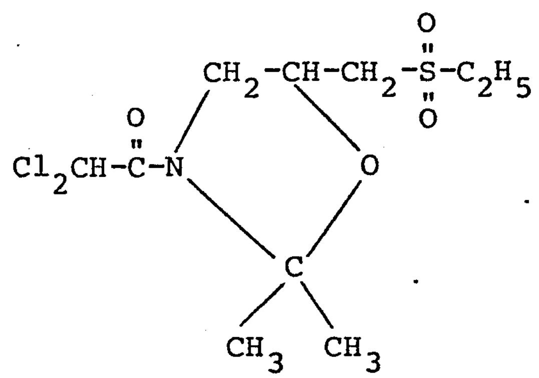

2. A process for the production of a compound as claimed in claim 1 characterised in that it comprises oxidising an appropriate oxazolidine.

3. A composition characterised in that it comprises a compound as claimed in claim 1 and an inert diluent or carrer and/or adjuvant.

4. A composition characterised in that it comprises a non-phytotoxic antidotally-effective amount of a compound as claimed in claim 1 and a herbicidally-effective amount of a thiocarbamate corresponding to the following general formula:

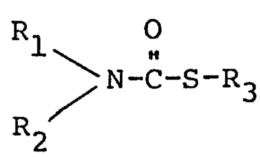

wherein

$R_1$ represents $C_1$-$C_6$ alkyl;

$R_2$ represents $C_1$-$C_6$ alkyl or cyclohexyl; or

$R_1$ and $R_2$ together represents $C_4$-$C_{10}$ alkylene; and
$R_3$ represents $C_1$-$C_6$ alkyl, $C_2$-$C_6$ alkenyl or benzyl, all of which may optionally be chloro-, bromo- or iodo-substituted.

5. A composition as claimed in claim 4 wherein:
$R_1$ represents propyl; $R_2$ represents propyl and $R_3$ represents propyl; or
$R_1$ represents propyl, $R_2$ represents propyl and $R_3$ represents ethyl; or
$R_1$ represents cyclohexyl; $R_2$ represents ethyl; and $R_3$ represents ethyl.

6. A method of establishing herbicidal selectivity characterised in that it comprises applying to a locus where the said selectivity is desired an antidotally-effective amount of a compound as claimed in claim 1 or a composition as claimed in claim 3 or a composition as claimed in claim 4 or claim 5.

7. The use of a compound as claimed in claim 1 as a herbicide antidote.

0074255

(Austria)

CLAIMS:

1. A process for the production of a compound corresponding to the following formula:

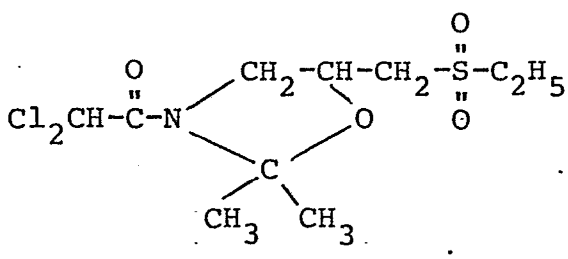

characterised in that it comprises oxidising an appropriate oxazolidine.

2. A process for the production of a composition characterised in that it comprises mixing a compound produced by a process as claimed in claim 1 with an inert diluent or carrier and/or adjuvant.

3. A process for the production of a composition characterised in that it comprises mixing a non-phytotoxic antidotally-effective amount of a compound produced by a process as claimed in claim 1 with a herbicidally-effective amount of a thiocarbamate correponding to the following general formula:

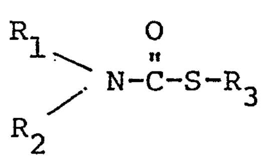

wherein

$R_1$ represents $C_1$-$C_6$ alkyl;

$R_2$ represents $C_1$-$C_6$ alkyl or cyclohexyl; or

$R_1$ and $R_2$ together represent $C_4$-$C_{10}$ alkylene; and

$R_3$ represents $C_1$-$C_6$ alkyl, $C_2$-$C_6$ alkenyl or benzyl, all of which may optionally be chloro- bromo- or iodo-substituted.

4. A process as claimed in claim 3 wherein:
$R_1$ represents propyl; $R_2$ represents propyl; and $R_3$ represents propyl; or
$R_1$ represents propyl; $R_2$ represents propyl; and $R_3$ represents ethyl; or
$R_1$ represents cyclohexyl, $R_2$ represents ethyl; and $R_3$ represents ethyl.

5. A method of establishing herbicidal selectivity characterised in that it comprises applying to a locus where the said selectivity is desired an antidotally-effective amount of a compound produced by a process as claimed in claim 1 or a composition produced by a process as claimed in claim 2 or a composition produced by a process as claimed in claim 3 or claim 4.

6. The use of a compound produced by a process as claimed in claim 1 as a herbicide antidote.

European Patent Office

# EUROPEAN SEARCH REPORT

0074255

Application number

EP 82 30 4641

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | EP-A-0 021 759 (STAUFFER)<br>*The whole document* | 1-7 | C 07 D 263/04<br>A 01 N 25/32 |
| | | | TECHNICAL FIELDS SEARCHED (Int. Cl. 3) |
| | | | C 07 D 263/00<br>A 01 N 25/00 |

The present search report has been drawn up for all claims

| Place of search | Date of completion of the search | Examiner |
|---|---|---|
| THE HAGUE | 22-11-1982 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82